(19) Europäisches Patentamt
European Patent Office
Office européen des brevets

(11) **EP 4 311 806 A1**

(12) **EUROPEAN PATENT APPLICATION**

(43) Veröffentlichungstag:
**31.01.2024 Patentblatt 2024/05**

(21) Anmeldenummer: **22187538.8**

(22) Anmeldetag: **28.07.2022**

(51) Internationale Patentklassifikation (IPC):
*C01B 3/36* (2006.01)          *C07C 2/78* (2006.01)
*C07C 11/24* (2006.01)          *C01B 3/00* (2006.01)

(52) Gemeinsame Patentklassifikation (CPC):
(C-Sets verfügbar)
**C01B 3/00; C01B 3/36; C07C 2/78**          (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **BASF SE**
**67056 Ludwigshafen am Rhein (DE)**

(72) Erfinder:
• **Vicari, Maximilian**
**67056 Ludwigshafen am Rhein (DE)**

• **Weichert, Christian**
**67056 Ludwigshafen am Rhein (DE)**
• **Britzius, Susanne**
**67056 Ludwigshafen am Rhein (DE)**
• **Reif, Wolfgang**
**67056 Ludwigshafen am Rhein (DE)**

(74) Vertreter: **BASF IP Association**
**BASF SE**
**GBI-C006**
**67056 Ludwigshafen (DE)**

(54) **VERFAHREN ZUR HERSTELLUNG VON ACETYLEN UND SYNTHESEGAS**

(57)    Vorgeschlagen wird ein Verfahren zur Herstellung von Acetylen und Synthesegas durch partielle Oxidation von Kohlenwasserstoffen mit Sauerstoff.

**EP 4 311 806 A1**

(52) Gemeinsame Patentklassifikation (CPC): (Forts.)

C-Sets
**C07C 2/78, C07C 11/24**

**Beschreibung**

Die vorliegende Erfindung betrifft ein Verfahren zur Herstellung von Acetylen und Synthesegas durch partielle Oxidation von Kohlenwasserstoffen mit Sauerstoff.

Die obige partielle Oxidation ist eine Hochtemperaturreaktion, die üblicherweise in einem Reaktorsystem, umfassend eine Mischeinrichtung, einen Brennerblock sowie eine Quencheinrichtung, durchgeführt wird, und beispielsweise in Ullmanns Encyclopedia of Industrial Chemistry (5th Edition, Volume A1, Seiten 97-144) oder US 005824834A beschrieben ist.

Gemäß Ullmanns Encyclopedia of Industrial Chemistry (5th Edition, Volume A1, Seiten 97-144) erfolgt die Aufheizung der Einsatzstoffe getrennt in Vorheizern. Als Einsatzstoffe werden hier leichte Kohlenwasserstoffe wie z.B. Methan, Ethan, Propan bis Hexan und deren Isomere und hoch angereicherter Sauerstoff (Sauerstoffgehalt größer als 95 vol.%) verwendet. Die aufgeheizten Einsatzstoffe werden in einer Mischeinrichtung gemischt und über einen Mischdiffusor einem Brenner und weiter einem Feuerraum zugeführt. Stromab des Feuerraums wird mittels Düsen ein wässriges Quenchmedium dem Spaltgas zugeführt und dieses schnell auf etwa 80 - 90 °C abgekühlt. Der Prozess wird durch geeignete Wahl der Sauerstoffzahl $\lambda$ so betrieben ($\lambda$ < 0,31), wobei man unter der Sauerstoffzahl $\lambda$ das Verhältnis aus der tatsächlich im zweiten Einsatzstrom vorhandenen Sauerstoffmenge zur stöchiometrisch notwendigen Sauerstoffmenge versteht, dass die Ausbeute an Acetylen bezogen, auf das trockene Spaltgas, optimal groß wird. So ist hier eine Acetylenkonzentration im trockenen Spaltgas von > 8 vol.% zu erreichen. Hierbei wird unter Sauerstoffzahl $\lambda$ wie üblich das Verhältnis aus der tatsächlich vorhandenen Sauerstoffmenge zur stöchiometrisch notwendigen Sauerstoffmenge verstanden, die für die vollständige Verbrennung der Einsatzstoffe erforderlich ist. Hierbei wird jedoch auch die Rußbeladung des Spaltgases maximal. Der im Feuerraum aus der Gasphase gebildete Ruß wird zum Teil durch den Quench, in einer anschließenden Kühlkolonne und einem daran anschließenden Elektrofilter abgeschieden. Der wertprodukthaltige Produktgasstrom wird getrennt über die Kühlkolonne abgeführt. Nach dem Elektrofilter ist die Rußkonzentration im restlichen Spaltgas auf etwa 1 mg/m$^3$ gesunken. Der im Prozesswasser aus dem Quench, der Kühlkolonne und dem Elektrofilter enthaltene Ruß besitzt einen hohen Kohlenwasserstoffanteil und ist daher hydrophob, was ihn auf dem Prozesswasser aufschwimmen lässt. Daher wird dieses rußbeladene Prozesswasser über sogenannte offene Rußrinnen mit Oberflächen-Partikelabscheidern geleitet. Die aufschwimmenden Rußanteile werden dabei abgetrennt und einer Feuerung zugeführt. Das so gereinigte Prozesswasser wird anschließend über einen offenen Kühlturm gefahren und somit abgekühlt.

Ein weiteres Verfahren zur Herstellung von Acetylen und Synthesegas durch partielle Oxidation von Kohlenwasserstoffen mit Sauerstoff ist in US 005824834A beschrieben. Dabei handelt es sich um ein Rußmengen-optimiertes, geschlossenes Wasserquenchverfahren, das mit einem mageren Feedstrom betrieben wird, und zwar mit einem Feedstrom mit einer Sauerstoffzahl $\lambda$ < 0,31. Das Verfahren hat jedoch den Nachteil einer reduzierten Ausbeute an Wertprodukt Acetylen. Der Acetylengehalt im Spaltgas liegt hier üblicherweise in einem Bereich von 5 vol.-%. bis 7 vol.%

Bei dieser Verfahrensvariante wird das wässrige Quenchmedium ebenfalls mittels Düsen dem Spaltgas zugeführt und dieses schnell auf etwa 80 -90 °C abgekühlt. Der im Feuerraum aus der Gasphase gebildete Ruß wird zum Teil durch den Quench, eine anschließende mit rezirkulierendem Wasser betriebene Kühlkolonne und einem daran anschließenden Elektrofilter und/oder Spaltgasverdichter abgeschieden. Der werthaltige Produktgasstrom wird getrennt über die Kühlkolonne abgeführt. Der Prozess wird hierbei durch Wahl der Sauerstoffzahl $\lambda$ so betrieben ($\lambda$ > 0,31), dass die anfallende Rußmenge im Spaltgas so gering ist, dass alleine durch die Ausschleusung des anfallenden Reaktionswassers aus der Verbrennung der stationäre Betrieb gewährleistet werden kann. Dadurch reduziert sich jedoch der Acetylengehalt im trockenen Spaltgas um 2 Prozentpunkte gegenüber dem vorstehend beschriebenen Verfahren auf etwa 6 Vol.-%. Damit wird eine geschlossene, also gegenüber der Umwelt abgetrennte Wasserquench-Fahrweise ermöglicht. Der Vorteil gegenüber der zuvor beschriebenen Verfahrensvariante ist somit die Möglichkeit des geschlossenen Betriebes ohne weitere Trennapparaturen.

Hier eine typische Erdgaszusammensetzung aufgeführt, welche als Einsatzstoff Verwendung findet:

|  | Erdgas |
|---|---|
| **Methan [Vol.%]** | 99,48% |
| **Ethan [Vol.%]** | 0,12% |
| **Kohlendioxid [Vol.%]** | 0,20% |
| **Stickstoff [Vol.%]** | 0,20% |

Ausgehend von einem derartig zusammengesetzten Erdgas ergibt sich bei einer Sauerstoffzahl $\lambda$ von 0,314 folgende Zusammensetzung des Spaltgases durch partielle Oxidation des Erdgases mit reinem Sauerstoff (99.7 Vol.%

$O_2$, 0,3 Vol.% $N_2$)

|  | Spaltgas (trocken) |
|---|---|
| **Wasserstoff [Vol.%]** | 58,959% |
| **Stickstoff [Vol.%]** | 0,215% |
| **Sauerstoff [Vol.%]** | 0,193% |
| **Kohlenmonoxid [Vol.%]** | 28,406% |
| **Kohlendioxid [Vol.%]** | 3,346% |
| **Methan [Vol.%]** | 2,505% |
| **Acetylen [Vol.%]** | 6,006% |
| **Ethylen [Vol.%]** | 0,205% |
| **Ethan [Vol.%]** | 0,005% |
| **Propin [Vol.%]** | 0,020% |
| **Propadien [Vol.%]** | 0,020% |
| **Vinylacetylen [Vol.%]** | 0,024% |
| **Diacetylen [Vol.%]** | 0,097% |

Das Gemisch wird im weiteren als Spaltgas bezeichnet.

Der Restsauerstoffgehalt von 0,157% und der Restmethangehalt von 2,505 Vol.% resultieren aus dem nicht vollständigen Umsatz bei der partiellen Oxidation.

Bei diesen Bedingungen, die auf eine optimale Acetylenausbeute beim Betrieb als geschlossener Wasserquench abgestimmt sind, werden auch weitere ungesättigte Komponenten gebildet. Neben Ethylen sind dies insbesondere Methylacetylen, Diacetylen, Propadien, 1,3-Butadien und Vinylacetylen. Diese Komponenten werden im weiteren höhere Acetylene bzw. als Gasstrom HA-Gas bezeichnet.

Das Spaltgas wird nach Abkühlung in der Kühlkolonne in 2-stufigen Schraubenverdichtern (a) mit Zwischenkühlung auf 10 bar(abs.) verdichtet.

Schraubenverdichter eignen sich hier, wegen ihrer robusten Funktionsweise und höherer Toleranz gegenüber Ablagerungen, besser als Turboverdichter.

Im Konzentrierungsabschnitt der Anlage erfolgt die Auftrennung des Spaltgasstroms in drei Ströme mittel N-Methylpyrrolidon (NMP) als Lösungsmittel:

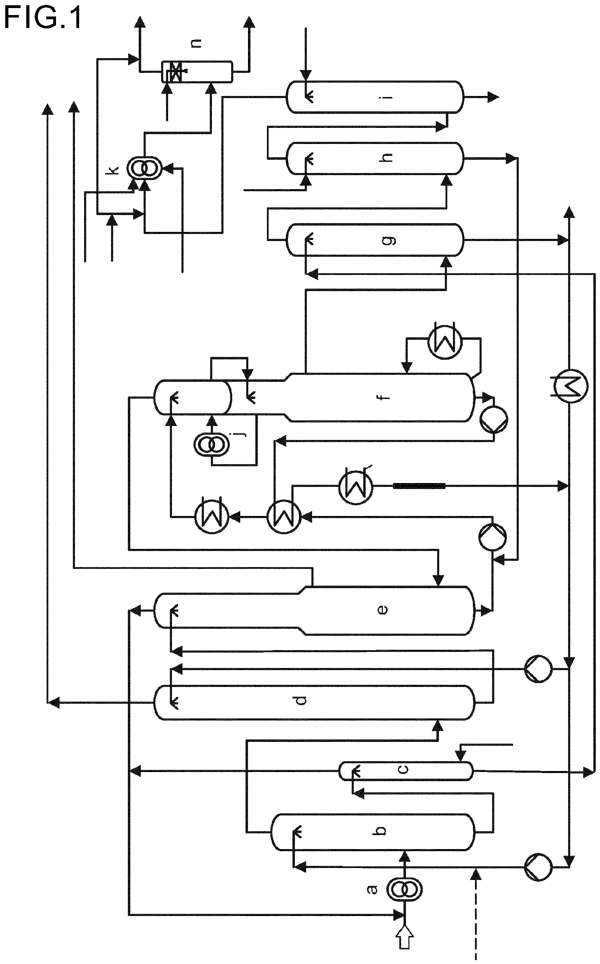

1. Höhere Acetylene (HA-Gas), die am besten in NMP löslichen Anteile des Spaltgases.
2. Acetylen als Produkt, das weniger löslich in NMP ist als die HA-Gas Komponenten, aber besser löslich als die übrigen Komponenten.
3. Roh Synthesegas, das aus den in NMP nur weit schlechter löslichen Komponenten wie $H_2$, CO, $CO_2$, Rest-$CH_4$, $C_2H_6$ und Rest-$O_2$ besteht.
4. Die hierfür erforderlichen Verfahrensschritte werden im Folgenden exemplarisch anhand der Figur 1 näher beschrieben.

In der Vorwäsche (b) werden aus dem verdichtete Spaltgas mit einer kleinen Menge N-Methylpyrrolidon (NMP), annähernd alle höheren Acetylene bis auf Vinylacetylen ausgewaschen. Im darauffolgenden Hauptwascher (d) erfolgt die Herauslösung des gesamt enthaltenen Acetylens, der Rest höheren Acetylene und eines Teils des $CO_2$ mit einer großen Menge NMP. Roh-Synthesegas verlässt den Hauptwascher (d) über Kopf. Das so beladene NMP wird in mehreren Stufen entgast, wobei der Druck abgesenkt und die Temperatur erhöht wird. Stufenweise wird der Druck auf 1,05 bar(abs.) bis 1,4 bar(abs.), bevorzugt auf 1,2 bar(abs.) bis 1,25 bar(abs.) und anschließend auf 0,1 bar(abs) bis 0,3 bar(abs) bevorzugt 0,19 bar(abs) bis 1,2 bar(abs) abgesenkt. Die Temperatur wird dabei stufenweise von 30°C bis 50°C, bevorzugt 36°C bis 40°C über 90°C bis 100°C, bevorzugt 95°C bis 97°C auf 110°C bis 130°C, bevorzugt 112°C bis 116°C erhöht. Der $CO_2$-Stripper (e) wird annähernd bei Umgebungsdruck und Temperatur betrieben. Im Gegenstrom zu dem Gasstrom aus den folgenden Entgasungsschritten wird das beladene NMP dem $CO_2$-Stripper (e) aufgegeben. Dabei entspannt sich das beladene NMP und der größte Teil des gelösten $CO_2$, als weniger lösliche Komponente, wird am Kopf des $CO_2$-Strippers (e) frei und anschließend ausgestrippt. Das freiwerdende $CO_2$ wird auf die Saugseite der Spaltgasverdichter (a) zurückgefahren und somit in das Roh-Synthesegas gezwungen. Acetylen verlässt den $CO_2$-Stripper (e) über eine gasförmigen Seitenabzug. Der Sumpfabzug, der noch einen Teil der gelösten Gase enthält, wird in 2 folgenden Stufe bei 110°C - 120°C erst bei Normaldruck, dann unter Vakuumbedingungen vollständig entgast. Die höheren Acetylene, wie Vinylacetylen, Methylacetylen und überschüssiges Prozesswasser wird als Seitenabzug aus der Vakuumkolonne (f) abgezogen. Die Kontrolle des Wassergehalts erfolgt über die Aufkochrate im Sumpf der Vakuumkolonne (f). Aus dem Sumpf der Vakuumkolonne (f) wird das vollständig entgaste NMP nach Abkühlung zur Vorwäsche (b) und Hauptwascher (d) zurückgefahren.

Der Sumpfablauf der Vorwäsche (b) enthält außer den höheren Acetylenen auch noch gelöstes Acetylen, das im Acetylenstripper (c) mit Roh-Synthesegas abgestrippt wird. Das Kopfprodukt des Acetylenstrippers (c), größtenteils Acetylen, wird auf die Saugseite der Spaltgasverdichter (a) zurückgefahren. Der Sumpfablauf enthält die höheren Acetylene, besonders Diacetylen, gelöst in NMP. Dieser Strom wird zusammen mit dem gasförmigen Seitenabzug der Vakuumkolonne (f), im Vakuumstripper (g) entgast. Das Kopfprodukt des Vakuumstrippers (g) enthält neben den höheren Acetylenen und Wasser auch noch gewisse Mengen an NMP. Das NMP wird in der Seitenkolonne (h) mit einer kleinen Menge an Wasser aus dem Gasstrom herausgewaschen und in den Lösemittelkreislauf zurückgefahren. Die Kondensation in der Kondensationskolonne (i) erfolgt in direktem Kontakt mit gekühltem Prozesswasser aus dem Prozesswasserkühlkreislauf. Damit kondensiert ein Großteil des enthaltenen Wasserdampfs. Die höheren Acetylene verlassen die Kondensationskolonne (i) über Kopf und werden mit Verdünnungsgas verdünnt um den kritischen Deflagrationsgrenzdruck zu unterschreiten

Der kritische Deflagrationsgrenzdruck des Gasgemisches errechnet sich wie folgt:

$$p_{krit.} = 1 / \Sigma ( x_i / p_{krit.i} )$$

$p_{krit.i}$ ist dabei der kritische Deflagrationsgrenzdruck der Einzelkomponente und $x_i$ der dazugehörige Molenbruch. Als Verdünnungsgas wird vorzugweise Erdgas verwendet, das im Vergleich zu Stickstoff oder auch dem roh-Synthesegas eine höhere spezifische Wärmekapazität aufweist.

Trotz Verdünnung weist das Gemisch aus den höheren Acetylenen immer noch eine ausgeprägte Tendenz zur Polymerisation auf. Diese Polymere könne sich selbst entzünden und sind reibe- und stoßempfindlich.

Die Verdichtung aus dem Vakuum auf leichten Überdruck (ca. 1,2 bar(abs.)) erfolgt im HA-Gasverdichter (k), vorzugsweise vom Typ Schraubenverdichter. Dieser Verdichter Typ gestattet es auch, Prozesswasser in den Zulauf einzuspritzen, um Anhaftungen von Polymeren zu verhindern und auch die adiabate Temperaturerhöhung, die durch die Verdichtung entsteht, zu verringern. Außerdem ist das Dichtungssystem mit Stickstoff beaufschlagt und gespült, um die Dichtflächen von Verunreinigungen und Polymerisaten aus dem HA-Gas Komponenten freizuhalten. Alle drei Maßnahmen führen zu Laufzeiten der HA-Gasverdichter (k) über mehrere Jahre. Ein Gemisch von Wassertröpfchen, Wasserdampf, Verdünnungsgas und HA-Gas verlässt den HA-Gas Verdichter auf der Druckseite. Um den kritischen Deflagrationsgrenzdruck deutlich zu unterschreiten, wird auch auf der Druckseite des HA-Gas Verdichters nach Kondensat Abscheidung bzw. Kühlung, nochmals Verdünnungsgas zugegeben.

Eine typische Anwendung für den verdichteten HA-Gasstrom ist die thermische Nutzung in den Vorheizern der Erdgas- und/oder Sauerstoffvorheizern der Acetylenerzeugung oder die direkte Unterfeuerung in einem Dampfkessel. Für der derartige Ströme mit Polymerisationsneigung eignet sich ein Sprühkondensator wie im VDI Wärmeatlas, Misch- und Sprühkondensation, beschrieben. (Hochberg, U., Grave, H. (2019). J4 Misch- und Sprühkondensation. In: Stephan, P., Kabelac, S., Kind, M., Mewes, D., Schaber, K., Wetzel, T. (eds) VDI-Wärmeatlas. Springer Reference Technik(). Springer Vieweg, Berlin, Heidelberg. https://doi.org/10.1007/978-3-662-52989-8 66) Bisher wurde der Strom mit gekühltem Prozesswasser in einem entsprechenden Sprühkondensator (n) im Gegenstrom heruntergekühlt und dann der thermischen Nutzung zugeführt.

Das abzukühlende HA-Gas wird dem unteren Teil Sprühkondensator (n) zugeführt.

Im Gegenstrom wird von oben Kühl-Prozesswasser zur Abkühlung eingespritzt. Im oberen Teil befindet sich auch ein Demister, um die durch die Verdüsung auch entstehenden feinen Tröpfchen zu koaleszieren und abzuscheiden und Richtung Sumpf des Sprühkondensator (n) abtropfen zu lassen.

Aus dem Sumpf wird dann das warme Prozesswasser abgezogen.

Im Sprühkondensator (n) kommt es auf Grund von Polymerisation von HA-Gas Komponenten zu Ablagerungen, die eine regelmäßige Abstellung und anschließende Reinigung erzwingen. Üblicherweise ist eine solche Abstellung zum Zwecke der Reinigung in Abständen von 4 Wochen bis 8 Wochen erforderlich. Dies bewirkt sowohl einen hohen Arbeitsaufwand wegen der Gefährlichkeit der Polymere und hohe Reinigungskosten. Die Polymerisation wird durch den hohen Gehalt an polymerisierbaren Komponenten, insbesondere das Diacetylen, im Prozesswasser, nach Kontakt mit dem HA-Gas, hervorgerufen. Dies kommt durch die Temperaturabhängigkeit der Löslichkeit der polymerisierbaren Komponenten im Prozesswasser zustande. Bei niedrigen Temperaturen, die im Fall der Abkühlung in einem Sprühkondensator vorliegen, kommt es zu einer hohen Konzentration von gelösten polymerisierbaren Komponenten im Prozesswasser, die eine Polymerisation massiv begünstigt.

In Figur 1 werden die folgenden Bezeichnungen verwendet:

a) Spaltgasverdichter
b) Vorwäscher
c) Acetylenstripper
d) Hauptwäscher
e) CO2 Stripper
f) Vakuumkolonne
g) Vakuumstripper
h) Seitenkolonne
i) Kondensationskolonne
j) Vakuumverdichter
k) K HA-Gas Verdichter
n) Sprühkondensator

Die vorliegende Erfindung, beschäftigt sich mit der Optimierung dieses Anlagenteils, um die, durch ablagerungsbedingte Verstopfungen der Anlage, erzwungenen häufigen Abstellung zu verhindern, die Sicherheit des Prozesses zu steigern und somit ein wirtschaftlicheres Verfahren zur Verfügung zu stellen. Durch das Wegfallen des Einspritzwassers ist das Verfahren deutlich einfacher und ist kostengünstiger. So ist kein Einspritzwasser mehr erforderlich so dass Einspritzung, Kühlung und Umpumpen entfällt. Außerdem wird der HA-Gasstrom wärmer der anschließenden thermischen Nutzung zugeführt, so dass sich dort auch die thermische Effizienz verbessert.

Die Aufgabe wird gelöst durch ein zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom

- a.) getrennt voneinander vorgeheizt,
  in einem Verhältnis der Massenströme des zweiten Einsatzstromes zum ersten Einsatzstrom entsprechend einer Sauerstoffzahl $\lambda$ von kleiner oder gleich 0,35, bevorzugt kleiner oder gleich 0,33 gemischt werden, wobei man unter der Sauerstoffzahl $\lambda$ das Verhältnis aus der tatsächlich im zweiten Einsatzstrom vorhandenen Sauerstoffmenge zur stöchiometrisch notwendigen Sauerstoffmenge versteht, die für die vollständige Verbrennung des einen oder der mehreren, im ersten Einsatzstrom enthaltenen Kohlenwasserstoffe, erforderlich ist,
- b.) über einen Brennerblock einem Feuerraum zugeführt werden, worin die partielle Oxidation der Kohlenwasserstoffe stattfindet,
- c.) unter Erhalt eines Spaltgases, das stromabwärts des Feuerraumes nach Abkühlung in einer Kühlkolonne einem Verdichter zugeführt wird und dort komprimiert wird
- d.) anschließend einem Vorwäscher zuleitet in welchem durch Zugabe von Lösungsmittel das zugeführte Spaltgas von großen Teilen der im Spaltgas enthaltenen aromatischen Komponenten und höheren Acetylenen abgetrennt wird und diese am Sumpf des Vorwäschers abführt,
- e.) den im Vorwäscher abgereicherten Spaltgasstrom am Kopf des Vorwäschers entnimmt und einem Hauptwäscher zuleitet, in welchem der zugeführte Strom im Gegenstrom zu einem am Kopf zugeführten Lösungsmittel weiter aufgetrennt wird, wobei am Sumpf des Hauptwäschers im wesentlichen Acetylen, Reste noch enthaltener höherer Acetylene sowie CO2 und Lösungsmittel abgeführt werden und über Kopf des Hauptwäschers im wesentlichen Roh-Synthesegas abgeführt wird,
- f.) den dem Hauptwäscher am Sumpf entnommenen Strom einem CO2 Stripper zuführt, in welchem im Gegenstrom zu zusätzlich eingeleitetem Lösungsmittel das enthaltene CO2 ausgestrippt wird und über Kopf des CO2 Strippers abgeleitet wird, Acetylen aus dem CO2 Stripper über einen Seitenabzug abgeführt wird und der verbleibende noch höhere Acetylene enthaltene Reststrom den CO2 Stripper am Sumpf verlasst,
- g.) dieser dem Sumpf des CO2 Strippers entnommene Strom in einer nachgeschalteten Vakuumkolonne entgast wird, wobei die noch enthaltenen höheren Acetylene und Prozesswasser über einen Seitenabzug der Vakuumkolonne abgezogen werden,
- h.) wobei der dem Sumpf des Vorwäschers entnommene Strom sowie der am Seitenabzug der Vakuumkolonne entnommene Strom einem Vakuumstripper zugeführt werden wobei am Kopf des Vakuumstrippers ein Strom entnommen wird, der vorwiegend höhere Acetylene enthält,
- i.) das im Kopfstrom der Vakuumkolonne noch enthaltene Lösungsmittel in einer nachgeschalteten Seitenkolonne durch Zugabe von Wasser herausgewaschen wird,
- j.) dem am Kopf der Seitenkolonne entnommenen Strom in einer nachgeschalteten Kondensationskolonne durch Abkühlung von Wasser befreit wird und über Kopf abgezogen wird,

- k.) der dem Kopf der Seitenkolonne entnommene Strom mittels eines Verdichters komprimiert wird,
- dadurch gekennzeichnet, dass

der komprimierte Strom einem gas/flüssig Abscheider zugeführt wird, wobei die im wesentlichen Wasser enthaltende flüssige Phase von der im wesentlichen höhere Acetylene enthaltenden gasförmigen Phase abgetrennt werden

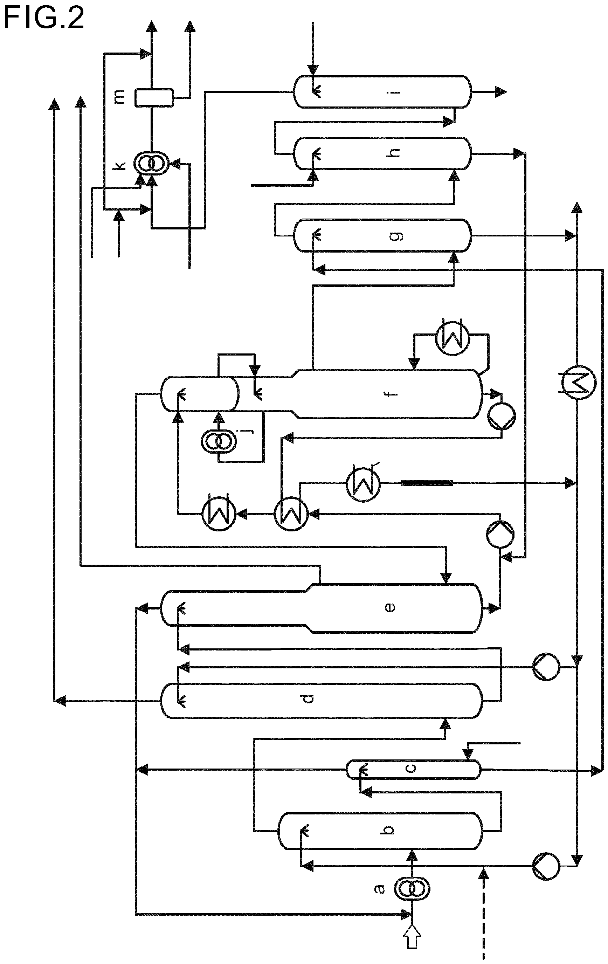

Erfindungsgemäß wird der Sprühkondensator (n) durch einen gas/flüssig-Abscheider, den Prozesswasserabscheider (m) ersetzt, um die Flüssigphase aus dem Gasstrom zu entfernen. Das erfindungsgemäße Verfahren ist in Figur 2 dargestellt, die Bezeichnungen der dargestellten Apparate sind hier analog zu Figur 1 zugeordnet. Die Flüssigphase kommt durch die Wassereinspritzung in dem vorgeschalteten HA-Gas-Verdichter (k) zustande. Der Gasstrom wird dann ohne weitere Kühlung und Wärmeverluste der thermischen Nutzung zugeführt. Ein großer Vorteil ist, dass die abgeschiedene Flüssigphase bei deutlich höherer Temperatur, wie bei dem Einsatz eines kühlenden Sprühkondensator (n) vorliegt und somit die Löslichkeit der polymerisierbaren Komponenten signifikant niedriger ist im Vergleich zum kühlenden Sprühkondensator (n). Durch den sehr niedrigen Gehalt der polymerisierbaren Komponenten wird die unerwünschte Polymerisation stark zurückgedrängt, dass Laufzeiten des erfindungsgemäßen gas/flüssig Abscheiders von mehr als einem Jahr erreicht werden können. Weiterhin können durch die erfindungsgemäße Ausgestaltung des Verfahrens Ressourcen eingespart werden, da kein zusätzliches Wasser hinzugefügt werden muss und auch energetisch betrachtet wird die Effektivität gesteigert.

Eine Abkühlung erfolgt nach Stand der Technik, um Kondensation in den folgenden Rohrleitungen und Prozessschritten zu verhindern. Ziemlich aufwändig wäre als Gegenmaßnahme eine effiziente Begleitbeheizung im anschließenden Rohrleitungssystem und den folgenden Apparaten.

Wenn der nächste Prozessschritt, hier die thermische Nutzung durch Verbrennung, optimal örtlich nahe aufgebaut ist, spielt dieser Nachteil keine Rolle.

Nach Stand der Technik können verschieden Abscheidertypen eingesetzt werden wie z.B. Umlenkabscheider, Fliehkraftabscheider, Filtrationsabscheider, Drahtgestrickabscheider, Lamellenabscheider oder Schüttungen.

Die erfindungsgemäß eingesetzten gas/flüssig Abscheider sind allgemein bekannt, sie sind beispielsweise in VDI-Richtlinie 3679 Blatt 3, August 2019 genauer beschrieben. Bevorzugt eignen sich insbesondere Fliekraftabscheider wegen ihres einfachen, kostengünstigen und robusten Aufbaus.

Vorteilhafterweise bietet das erfindungsgemäße Verfahren die Möglichkeit, die Abtrennung der flüssigen Phase von der Gasphase im erfindungsgemäß eingesetzten gas/flüssig Abscheider bei deutlich höheren Temperaturen durchzuführen als gemäß dem Stand der Technik. Während bei Verwendung eines Sprühkondensators die Temperaturen bei der Abtrennung bei etwa 25°C bis. 65°C liegen, kann die Abtrennung beim erfindungsgemäßen Verfahren so durchgeführt werden, dass die Temperatur der den gas/flüssig Abscheider verlassenden flüssigen Phase von 70°C bis 100°C, bevorzugt 70°C bis 90°C, besonders bevorzugt 84°C bis 85°C beträgt. Hierdurch kann wie vorstehend erläutert, die unerwünschte Polymersiation nachhaltig verringert werden.

Für die unerwünschte Polymerisation in der flüssigen Phase ist insbesondere das im Prozesswasser (flüssige Phase) enthaltene Diacetylen verantwortlich. Bei dem Verfahren gemäß Stand der Technik liegt der Gehalt dieser Komponente in etwa bei 0,1 Gew.% bis 0,2 Gew.%, bei dem erfindungsgemäßen Verfahren kann dieser Gehalt vorteilhafterweise auf 0,02 Gew.% bis 0,03 Gew.%., bevorzugt 0,03 Gew.% bis 0,04 Gew.%, besonders bevorzugt 0,033 Gew.% bis 0,035 Gew.% verringert werden.

| HA-Gas Verdichtung | |
| --- | --- |
| HA-Gas aus Kondensationskolonne (i) | Strom (l) |
| Nm3/t Acetylen | 48 |
| Temperatur [°C] | 40,2 |
| Druck [bar(abs.)] | 0,120 |
| Zusammensetzung [Vol.%] | |
| $CO_2$ | 0,499% |
| CO | 0,102% |
| $O_2$ | 0,111% |
| $N_2$ | 0,417% |
| $H_2$ | 0,196% |
| $CH_4$ | 0,034% |

(fortgesetzt)

| HA-Gas Verdichtung | |
|---|---|
| **HA-Gas aus Kondensationskolonne (i)** | **Strom (I)** |
| Acetylen | 1,813% |
| Propadien | 0,002% |
| Methylacetylen | 0,067% |
| Vinylacetylen | 6,003% |
| Diacetylen | 27,000% |
| Wasser | 63,756% |
| **Verdünnungsgas zu HA-Gas Verdichter (k)** | **Strom (II)** |
| Nm3/t Acetylen | 51 |
| Temperatur [°C] | 68,0 |
| Druck [bar(abs.)] | 5,000 |
| Zusammensetzung [Vol.%] | |
| $CO_2$ | 0,200% |
| $N_2$ | 0,200% |
| $CH_4$ | 99,480% |
| Ethan | 0,120% |
| **Einspritzwasser zu HA-Gas Verdichter (k)** | **Strom (III)** |
| kg/t Acetylen | 78 |
| Temperatur [°C] | 40,0 |
| Druck [bar(abs.)] | 5,700 |
| Zusammensetzung [Gew.%] | |
| Wasser | 100% |
| **Stickstoff zum HA-Gasverdichter (k)** | **Strom (IV)** |
| Nm3/t Acetylen | 9 |
| Temperatur [°C] | 19,0 |
| Druck [bar(abs.)] | 11,0 |
| Zusammensetzung [Vol.%] | |
| $N_2$ | 100,0% |
| **Druckseite HA-Gas Verdichter (k)** | **Strom (V)** |
| Nm3/t Acetylen | 205 |
| Temperatur [°C] | 84,7 |
| Druck [bar(abs.)] | 1,220 |
| Vol.% Wasser in der Gasphase | 47,0% |
| Gew.% Gasphase | 76,1% |

**Vergleichsbeispiel**

Das HA-Gas (I), 48 $Nm^3/t_{Acetylen}$ nach der Kondensationskolonne I, liegt bei 40°C und 0,120 bar(abs.) vor.

Um bei der Verdichtung den kritischen Deflagrationsgrenzdruck des HA-Gases nicht zu überschreiten, wird vorgewärmtes Verdünnungsgas (II) bereits auf der Saugseite des HA-Gas-Verdichter (k) zugegeben. Hier ist es 51 Nm$^3$/t$_{Acetylen}$ Erdgas bei 68°C.

Im HA-Gas-Verdichter (k) wird das HA-Gas (I) auf 1,22 bar(abs.) verdichtet, um es einer Nutzung über Atmosphärendruck zuzuführen. Die Verdichtung im HA-Gas-Verdichter (k), vom Typ Schraubenverdichter, erfolgt unter Wassereinspritzung (III) von 78 kg/t$_{Acetylen}$. Das Dichtungssystem wird mit Stickstoff beaufschlagt und gespült. Dabei gelangen 9 Nm$^3$/t$_{Acetylen}$ Stickstoff in den zu verdichtenden HA-Gas Strom. Auf der Druckseite des HA-Gas-Verdichters (k) hat das verdichte HA-Gas (V) eine Temperatur von 84,7°C und einen Gasgehalt von 76,1 Gew.%. Der Rest von 23,9% ist das nicht verdampfte Einspritzwasser als feine Tröpfchen.

| Vergleichsbeispiel mit Sprühkondensator (n) | |
|---|---|
| gekühltes HA-Gas vom Kopf Kühlkolonne (k) | Strom (VI) |
| Nm3/t Acetylen | 83 |
| Temperatur [°C] | 42,0 |
| Druck [bar(abs.)] | 1,200 |
| Zusammensetzung [Vol.%] | |
| CO2 | 0,407% |
| CO | 0,059% |
| O2 | 0,064% |
| N2 | 11,723% |
| H2 | 0,113% |
| CH4 | 62,145% |
| Acetylen | 1,027% |
| Propadien | 0,001% |
| Methylacetylen | 0,038% |
| Vinylacetylen | 3,406% |
| Diacetylen | 14,016% |
| Wasser | 7,001% |
| Sumpfabzug Kühlkolonne (k) | Strom (VII) |
| kg/t Acetylen | 1.652 |
| Temperatur [°C] | 62,0 |
| Druck [bar(abs.)] | 1,210 |
| Zusammensetzung [Gew. %] | |
| CO2 | 0,000% |
| CO | 0,000% |
| O2 | 0,000% |
| N2 | 0,000% |
| H2 | 0,000% |
| CH4 | 0,001% |
| Acetylen | 0,001% |
| Propadien | 0,000% |
| Methylacetylen | 0,000% |
| Vinylacetylen | 0,000% |
| Diacetylen | 0,178% |

(fortgesetzt)

| Vergleichsbeispiel mit Sprühkondensator (n) | |
|---|---|
| **gekühltes HA-Gas vom Kopf Kühlkolonne (k)** | **Strom (VI)** |
| Wasser | 99,819% |
| **Kühl-Prozesswasser zum Sprühkondensator (n)** | **Strom (VIII)** |
| kg/t Acetylen | 1.551 |
| Temperatur [°C] | 40,0 |
| Druck [bar(abs.)] | 5,700 |
| Zusammensetzung [Gew.%] | |
| Wasser | 100% |

Anschließend gelangt der verdichtete HA-Gas-Strom (V) in den unteren Teil des Sprühkondensator (n) zur Abkühlung. Von oben wird im Sprühkondensator(n) 1551 kg/t$_{Acetylen}$ Kühl-Prozesswasser (VIII) bei 40°C im Gegenstrom zum abzukühlenden HA-Gas eingespritzt. Dabei kühlt sich das HA-Gas (VI) auf 42°C ab. Dabei verringert sich auch der Dampfgehalt in der Gasphase von 47,3 Vol.%, [Vergleichsbeispiel (VI)] auf 7 Vol.% (VI). Aus dem Sumpf des Sprühkondensator (n) wird das Prozesswasser (VII) mit 62°C abgezogen. Aufgrund der niedrigen Temperatur lösen sich 0,178 Gew.% Diacetylen im Prozesswasser, das besonders hohe Polymerisationsneigung zeigt.

Auf Grund der Polymerablagerungen und damit einhergehender Verstopfung kann der Apparat nur ca. 6 Wochen betrieben werden, dann ist eine Reinigung erforderlich.

Erfindungsgemäßes Beispiel

Das HA-Gas (I), 48 Nm$^3$/t$_{Acetylen}$ nach der Kondensationskolonne I, liegt bei 40°C und 0,120 bar(abs.) vor.

Um bei der Verdichtung den kritischen Deflagrationsgrenzdruck des HA-Gases nicht zu überschreiten, wird vorgewärmtes Verdünnungsgas (II) bereits auf der Saugseite des HA-Gas-Verdichter (k) zugegeben. Hier ist es 51 Nm$^3$/t$_{Acetylen}$ Erdgas bei 68°C.

Im HA-Gas-Verdichter (k) wird das HA-Gas (I) auf 1,22 bar(abs.) verdichtet, um es einer Nutzung über Atmosphärendruck zuzuführen. Die Verdichtung im HA-Gas-Verdichter (k), vom Typ Schraubenverdichter, erfolgt unter Wassereinspritzung (III) von 78 kg/t$_{Acetylen}$. Das Dichtungssystem wird mit Stickstoff beaufschlagt und gespült. Dabei gelangen 9 Nm$^3$/t$_{Acetylen}$ Stickstoff in den zu verdichtenden HA-Gas Strom. Auf der Druckseite des HA-Gas-Verdichters (k) hat das verdichte HA-Gas (V) eine Temperatur von 84,7°C und einen Gasgehalt von 76,1 Gew.%. Der Rest von 23,9% ist das nicht verdampfte Einspritzwasser als feine Tröpfchen.

| Erfindungsgemäßes Beispiel | |
|---|---|
| **HA-Gas aus Prozesswasserabscheider (n)** | **Strom (VI)** |
| Nm3/t Acetylen | 148 |
| Temperatur [°C] | 84,7 |
| Druck [bar(abs.)] | 1,220 |
| Zusammensetzung [Vol.%] | |
| CO2 | 0,229% |
| CO | 0,033% |
| O2 | 0,036% |
| N2 | 6,522% |
| H2 | 0,063% |
| CH4 | 34,584% |
| Acetylen | 0,580% |
| Propadien | 0,000% |

(fortgesetzt)

| Erfindungsgemäßes Beispiel | |
| --- | --- |
| **HA-Gas aus Prozesswasserabscheider (n)** | **Strom (VI)** |
| Methylacetylen | 0,021% |
| Vinylacetylen | 1,921% |
| Diacetylen | 8,638% |
| Wasser | 47,373% |
| **Flüssiganfall Prozesswasserabscheider (n)** | **Strom (VII)** |
| kg/t Acetylen | 46 |
| Temperatur [°C] | 84,7 |
| Druck [bar(abs.)] | 1,220 |
| Zusammensetzung [Gew.%] | |
| $CO_2$ | 0,000% |
| CO | 0,000% |
| $O_2$ | 0,000% |
| $N_2$ | 0,000% |
| $H_2$ | 0,000% |
| $CH_4$ | 0,001% |
| Acetylen | 0,000% |
| Propadien | 0,000% |
| Methylacetylen | 0,000% |
| Vinylacetylen | 0,000% |
| Diacetylen | 0,034% |
| Wasser | 99,965% |

Anstatt das HA-Gas abzukühlen, wird im Prozesswasserabscheider (m) ohne Abkühlen nur das nicht verdampfte Einspritzwasser abgeschieden. Sowohl flüssige Phase, wie auch die Gasphase, haben eine Temperatur von 84,7°C. Die Gasphase, das verdichtet HA-Gase wird der weiteren Nutzung zugeführt. Auf Grund der deutlich höheren Temperatur von 84,7°C im Vergleich zum Vergleichsbeispiel von 62°C löst sich deutlich weniger der polymerisieranfälligen HA-Gas Komponenten in der wässrigen Flüssigphase des Prozesswassers. Bei 84,7°C ergibt sich ein Diacetylen-Gehalt im Prozesswasser von nur 0,034 Gew.%. Beim Vergleichsbeispiel sind es 0,176 Gew.% also ein Faktor von 5,2. Bedingt durch die geringere Konzentration kommt es im Prozesswasserabscheider (m) zu keiner keine störenden Abscheidungen von Polymerisaten, die eine Reinigung erzwingen würden. So kann der Prozesswasserabscheider (m) deutlich länger als 1 Jahr betrieben werden, während bei dem Verfahren gemäß Stand der Technik der dort verwendete Sprühkondensator in etwa alle 6 Wochen Zum Zwecke der Reinigung abgestellt werden muss.

**Patentansprüche**

1. Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom

   - a.) getrennt voneinander vorgeheizt,
   in einem Verhältnis der Massenströme des zweiten Einsatzstromes zum ersten Einsatzstrom entsprechend einer Sauerstoffzahl λ von kleiner oder gleich 0,35, bevorzugt kleiner oder gleich 0,33 gemischt werden, wobei man unter der Sauerstoffzahl λ das Verhältnis aus der tatsächlich im zweiten Einsatzstrom vorhandenen Sauerstoffmenge zur stöchiometrisch notwendigen Sauerstoffmenge versteht, die für die vollständige Verbrennung des einen oder der mehreren, im ersten Einsatzstrom enthaltenen Kohlenwasserstoffe, erforderlich ist,

- b.) über einen Brennerblock einem Feuerraum zugeführt werden, worin die partielle Oxidation der Kohlenwasserstoffe stattfindet,

- c.) unter Erhalt eines Spaltgases, das stromabwärts des Feuerraumes nach Abkühlung in einer Kühlkolonne einem Verdichter zugeführt wird und dort komprimiert wird

- d.) anschließend einem Vorwäscher zuleitet in welchem durch Zugabe von Lösungsmittel das zugeführte Spaltgas von großen Teilen der im Spaltgas enthaltenen aromatischen Komponenten und höheren Acetylenen abgetrennt wird und diese am Sumpf des Vorwäschers abführt,

- e.) den im Vorwäscher abgereicherten Spaltgasstrom am Kopf des Vorwäschers entnimmt und einem Hauptwäscher zuleitet, in welchem der zugeführte Strom im Gegenstrom zu einem am Kopf zugeführten Lösungsmittel weiter aufgetrennt wird, wobei am Sumpf des Hauptwäschers im wesentlichen Acetylen, Reste noch enthaltener höherer Acetylene sowie CO2 und Lösungsmittel abgeführt werden und über Kopf des Hauptwäschers im wesentlichen Roh-Synthesegas abgeführt wird,

- f.) den dem Hauptwäscher am Sumpf entnommenen Strom einem CO2 Stripper zuführt, in welchem im Gegenstrom zu zusätzlich eingeleitetem Lösungsmittel das enthaltene CO2 ausgestrippt wird und über Kopf des CO2 Strippers abgeleitet wird, Acetylen aus dem CO2 Stripper über einen Seitenabzug abgeführt wird und der verbleibende noch höhere Acetylene enthaltene Reststrom den CO2 Stripper am Sumpf verlasst,

- g.) dieser dem Sumpf des CO2 Strippers entnommene Strom in einer nachgeschalteten Vakuumkolonne entgast wird, wobei die noch enthaltenen höheren Acetylene und Prozesswasser über einen Seitenabzug der Vakuumkolonne abgezogen werden,

- h.) wobei der dem Sumpf des Vorwäschers entnommene Strom sowie der am Seitenabzug der Vakuumkolonne entnommene Strom einem Vakuumstripper zugeführt werden wobei am Kopf des Vakuumstrippers ein Strom entnommen wird, der vorwiegend höhere Acetylene enthält,

- i.) das im Kopfstrom der Vakuumkolonne noch enthaltene Lösungsmittel in einer nachgeschalteten Seitenkolonne durch Zugabe von Wasser herausgewaschen wird,

- j.) dem am Kopf der Seitenkolonne entnommenen Strom in einer nachgeschalteten Kondensationskolonne durch Abkühlung von Wasser befreit wird und über Kopf abgezogen wird,

- k.) der dem Kopf der Seitenkolonne entnommene Strom mittels eines Verdichters komprimiert wird,

- **dadurch gekennzeichnet, dass**

- der komprimierte Strom einem gas/flüssig Abscheider zugeführt wird, wobei die im wesentlichen Wasser enthaltende flüssige Phase von der im wesentlichen höhere Acetylene enthaltenden gasförmigen Phase abgetrennt werden.

2. Verfahren nach Anspruch 1, **dadurch gekennzeichnet, dass** die Auftrennung im gas/flüssig Abscheider bei einer Temperatur von 70°C bis 90°C erfolgt.

3. Verfahren nach einem der Ansprüche 1 bis 2, **dadurch gekennzeichnet, dass** die Auftrennung in dem gas/flüssig Abscheider ohne zusätzliche Zufuhr eines Kühlmediums erfolgt.

4. Verfahren nach einem der Ansprüche 1 bis 3, **dadurch gekennzeichnet, dass** der dem Kopf der Kopf der Seitenkolonne entnommene Strom nach seiner Verdichtung unmittelbar dem gas/flüssig Abscheider zugeführt wird.

5. Verfahren nach einem der Ansprüche 1 bis 4, **dadurch gekennzeichnet, dass** die im gas/flüssig Abscheider abgetrennte flüssige Phase einen Gehalt an Diacetylen von weniger als 0,1 Gew% aufweist.

6. Verfahren nach einem der Ansprüche 1 bis 5, **dadurch gekennzeichnet, dass** man als gas/flüssig Abscheider Umlenkabscheider, Fliekraftabscheider Lamellenabscheider oder Abscheider mit Schüttungen einsetzt.

FIG.1

# FIG.2

Europäisches
Patentamt

European
Patent Office

Office européen
des brevets

## EUROPEAN SEARCH REPORT

Application Number

EP 22 18 7538

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 5 824 834 A (BACHTLER MICHAEL [DE] ET AL) 20 October 1998 (1998-10-20) <br> * examples 1-7 * <br> * claims 1-26 * <br> ----- | 1-6 | INV. <br> C01B3/36 <br> C07C2/78 <br> C07C11/24 <br> C01B3/00 |

TECHNICAL FIELDS
SEARCHED      (IPC)

C07C
C01B

The present search report has been drawn up for all claims

| Place of search | Date of completion of the search | Examiner |
|---|---|---|
| The Hague | 9 January 2023 | Delanghe, Patrick |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...........................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 18 7538

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2023

| Patent document cited in search report | | Publication date | Patent family member(s) | | Publication date |
|---|---|---|---|---|---|
| US 5824834 | A | 20-10-1998 | CN | 1155532 A | 30-07-1997 |
| | | | FR | 2740131 A1 | 25-04-1997 |
| | | | IT | MI962020 A1 | 02-04-1998 |
| | | | RU | 2133728 C1 | 27-07-1999 |
| | | | UA | 48131 C2 | 15-08-2002 |
| | | | US | 5824834 A | 20-10-1998 |

EPO FORM P0459

For more details about this annex : see Official Journal of the European Patent Office, No. 12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 005824834 A

**In der Beschreibung aufgeführte Nicht-Patentliteratur**

- Ullmanns Encyclopedia of Industrial Chemistry. vol. A1, 97-144

- J4 Misch- und Sprühkondensation. **HOCHBERG, U. ; GRAVE, H.** VDI-Wärmeatlas. Springer Vieweg, 2019